(19) Europäisches Patentamt
European Patent Office
Office européen des brevets

(11) EP 1 356 802 A1

(12) EUROPEAN PATENT APPLICATION

(43) Date of publication:
**29.10.2003 Bulletin 2003/44**

(51) Int Cl.[7]: **A61K 7/06**, D06M 15/643

(21) Application number: **03252204.7**

(22) Date of filing: **08.04.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **22.04.2002 GB 0209131**

(71) Applicant: **THE PROCTER & GAMBLE COMPANY
Cincinnati, Ohio 45202 (US)**

(72) Inventors:
- **Glenn, Robert Wayne
Virginia Water, Surrey GU25 4HZ (GB)**
- **Godfrey, Simon Paul
Uxbridge, Middlesex UB8 1AR (GB)**
- **McMeekin, Anthony
Bagshot, Surrey GU19 5QY (GB)**
- **Boumard, Coralie Claude Monique
Egham, Surrey TW20 8HG (GB)**
- **Bureiko, Andrei Sergeevich
ascot, Berkshire SL5 0BB (GB)**

(74) Representative: **Kohol, Sonia et al
Patent Department,
Procter & Gamble Technical Centers Limited,
Rusham Park,
Whitehall Lane
Egham, Surrey TW20 9NW (GB)**

(54) **Fiber treatment blend**

(57) A fiber treatment composition is presented comprising a blend of organomodified silicones which deposits more evenly and durably than prior art conditioners on a viariety of fibers, especially hair, of differing levels of damage. This is achieved by operating with organomodified silicones within a defined hydrophilicty range.



Printed by Jouve, 75001 PARIS (FR)

## Description

FIELD OF THE INVENTION

The present invention relates to topical compositions for treating natural and synthetic fibrous substrates. The topical compositions comprise functionalized silicone blends having defined physico-chemical properties that exhibit superior conditioning efficacy on both polar and non-polar fibrous substrates than previously known silicone based conditioners, especially where the substrate is hair that has been previously damaged through chemical treatments, such as occurs during permanent dyeing, bleaching and permanent waving.

BACKGROUND OF THE INVENTION

Oxidative dyeing, otherwise known as permanent coloring leads to irreversible physico-chemical changes to the hair. Typically, during this process, two components are mixed together prior to application to the hair. These components usually comprise an oxidising agent, such as hydrogen peroxide, and a dyeing material, such as oxidative dye precursors and couplers (buffered at a high pH, typically around 10). After contacting with the hair, the mixture is left for a period of time suitable to allow the required color transformation to occur, after which the hair becomes more hydrophilic versus non-colored hair due to irreversible chemical changes. While not wishing to be bound by theory, this change in hair hydrophilicity appears to be due, among other things, to the oxidation of the keratin-keratin cystine amino acids within the hair creating more hydrophilic cysteic acid amino acid residues and the removal by hydrolysis of nature's hydrophobic F-Layer. This coloring process is usually repeated regularly by consumers in order to maintain their desired hair color and color intensity and also to ensure that new hair growth has the same color as the older hair. As a consequence the hair changes polarity from a relatively hydrophobic surface near the scalp where it could be experiencing its first color, to a progressively more polar substrate at the hair tips, which may have been subjected to multiple coloring treatments. A discussion of oxidation dyeing of hair can be found in "The Science of Hair Care" by Charles Zviak, Marcel Dekker, New York, 1986.

These irreversible physicochemical changes can also manifest themselves as increased roughness, brittleness and dryness leading to less manageable hair. The use of conditioners within the coloring process is known. Conditioning materials can be added to the colorant product, or alternatively these can be supplied within the colorant kit as a separate conditioner, and can thereby be applied to the hair either during the coloring event or after the colorant has been rinsed. As described in EP 0 275 707, it is known to use aminosilicone for this purpose. However, it has also been established that, in the case of more polar hair, such as that obtained after successive oxidative colorings, aminosilicone deposition is greatly reduced and cannot provide the same level of benefit in hair condition as for non-oxidatively colored hair, especially when delivered within the harsh coloring environment. Without wishing to be bound by theory, the reason for this may be that there exists a surface energy incompatibility between the polar chemically damaged hair with the relatively non-polar aminosilicone leading to poorer adhesion.

Improving deposition evenness between more and less damaged hair represents a major improvement area to enable sufficient deposition at the more polar/damaged tips for a noticeable conditioning benefit where it is needed most without over-depositing at the roots. Moreover, this technical challenge represents an even bigger issue across differing consumer populations. For instance, the differing damage levels found in different individuals can result in the same silicone generating acceptable deposition in some cases, but over-deposition, with the accompanying negative sensory implications, in others (e.g. first time colorers vs. frequent colorers, brunettes vs. bleached blondes etc). Hence, a silicone active that deposits evenly across all hair types and damage levels is highly desirable.

Improving hair feel immediately after coloring is not the only desirable property of a colorant conditioner. After the coloring process human hair becomes soiled due to its contact with the surrounding environment and from the sebum secreted from the scalp. This soiling of the hair causes it to have a dirty feel and unattractive appearance and necessitates shampooing with frequent regularity. Shampooing cleans the hair by removing excess soil and sebum, but can leave the hair in a wet, tangled, and generally unmanageable state. Once the hair dries, it is often left in a dry, rough, lustreless, or frizzy condition due to the removal of the hair's natural oils and other natural or deposited conditioning and moisturizing components. Hair can also be left with increased levels of static upon drying which can interfere with combing and result in a condition commonly referred to as "fly-away-hair". These conditions tend to be exaggerated on hair which has been previously oxidatively colored.

A variety of approaches have been developed to alleviate these post-shampoo problems. These approaches range from post-shampoo application of hair conditioners such as leave-on or rinse-off products, to hair conditioning shampoos which attempt to both cleanse and condition the hair from a single product. Hair conditioners are typically applied in a separate step following shampooing. The hair conditioners are either rinsed-off or left-on, depending upon the type of product used. Polydimethylsiloxanes (PDMS) are often employed as conditioning materials in both shampoo and conditioner applications to improve hair feel. However, it is known that, in the case of more hydrophilic hair obtained

after oxidative coloring, PDMS deposition is greatly reduced, and cannot provide the same benefit in hair condition as for non-oxidatively colored hair.

Creating a conditioner that does not need to be applied every time the hair is washed would be highly advantageous and is not something that prior art compositions, including compositions defined in the above-mentioned art, currently allow. This is especially the case for the hydrophilic oxidatively damaged hair, typical of hair that has been permanently colored, which is much more vulnerable to subsequently further damage during the routine shampooing process. Having a durably deposited conditioner would enable round-the-clock protection to the hair. It is therefore highly desired that a conditioning active deposited during the coloring process remains on the hair during washing cycles in the days and weeks following coloring to provide a durable conditioning benefit.

Lastly, to obtain an improved conditioning effect, it is also important to ensure that enough silicone fluid deposits on each filament to meet consumer needs, i.e. that the absolute deposition of silicone fluid is sufficient for this purpose, both initially and long-term after subsequent shampooings.

Summarising some of the consumer needs discussed above, a dilemma faced by the present inventors was to produce a conditioner that deposits evenly and durably on hair of differing damage states, from undamaged, virgin hair, at the one extreme, to hair exposed to multiple oxidative dye treatments, at the other.

Attempts appear to have been made in the prior art to solve some of the problems discussed above. To be more specific, there has been a move away from using the highly hydrophobic PDMS-based silicones and towards using functionalized silicones, comprising functional groups such as amines, discussed above, and (among others) quaternary ammonium moieties. US 6,136,304, for example, discusses problems associated with conditioning compounds which are too easily rinsed from the hair. It also discusses application of conditioners to both virgin and damaged hair. The ethoxylated quaternary ammonium functionalized silicones proposed to achieve these aims, such as ABIL-QUAT 3272 (see Table 1, below) and ABIL-QUAT 3270 (both having the CTFA designation of quaternium-80), produced by the Goldschmidt Chemical Corporation, Hopewell, Va, are so hydrophilic, however, that they are rapidly washed off during subsequent shampooings. In other words, they do not achieve sufficient durability to meet consumer needs. In terms of polarity, these silicones are at the other end of the spectrum from the PDMS-type materials, but they are similarly non-durable and therefore unsuitable.

A further approach known in the art is to employ blends of silicones. US 6,171,515, for example, teaches a blend of amine-, polyol-functionalized silicone and an epoxy-, glycol siloxane. The epoxy functionalises siloxane is much too hydrophilic for the present purposes and can act almost as a surfactant to pull the other, more durable silicone from any substrate it is deposited on, thereby destroying overall durability.

With the above discussion in mind, the invention will ideally provide a hair treatment composition comprising a functionalized silicone conditioning agent which deposits evenly on all types of hair which occur in today's human population, from undamaged, virgin hair, at the one extreme, to hair exposed to multiple oxidative dye treatments, at the other.

Additionally, the invention will ideally provide a hair treatment composition comprising a functionalized silicone conditioning agent which deposits evenly over the whole length of a hair strand, including both lengths of uncolored scalp hair and hair previously colored with an oxidative colorant.

Additionally, the present invention will ideally provide a hair treatment composition comprising a durable silicone-conditioning agent for use on oxidation dyed hair, which does not wash off so rapidly that the conditioning benefit is lost to the consumer.

These and other features, aspects, and advantages of the present invention will become evident to those skilled in the art from a reading of the present disclosure.

## SUMMARY OF THE INVENTION

According to a first aspect of the invention, a fiber treatment composition is presented, comprising a blend of a hydrophilic functionalized silicone having a hydrophilicity index (HI) value of greater than or equal to 85 and less than 100, and a hydrophobic functionalized silicone having an interfacial tension (IFT) of less than or equal to 15mN/m and an HI of less than 95, wherein the hydrophilic silicone and the hydrophobic silicone are immiscible with one another.

The functionalized silicone polymers according to the invention are capable of depositing evenly and durably on hair in all states of damage.

As used herein, the term "fiber" includes strands of natural or synthetic materials. Non-limiting examples of natural materials are amino acid based materials, including protinaceous materials such as wool, human hair, including velus hair, and animal fur; cotton; cellulose and silk. Non-limiting examples of synthetic materials are polyester, nylon and rayon.

As used herein, the term "functionalized" silicone includes polydimethylsiloxanes (PDMS) in which at least one methyl group has been replaced by a different group, which is preferably not hydrogen. The term "functional silicone" is synonymous with the term "functionalized silicone"

The hydrophilic and hydrophobic functional silicone components are immiscible wih one another - e.g., when intermixed at a 50:50 ratio within a clean beaker that is void of other components, the two functional silicone components are unable to remain uniformly mixed or blended with another after the mixing energy is removed creating two distinct and visible phases after three weeks standing under ambient temperature and pressure conditions.

The terms "interfacial tension" and "hydrophilicity index" are to be understodd as defined hereinbelow.

As used herein, the term "even" when used in relation to functionalized silicone deposition refers to the relative deposition on damaged as opposed to undamaged hair. Phrases such as "deposits evenly" and "even deposition" are to be interpreted accordingly.

As used herein, the term "durable" used in relation to functionalized silicone deposition means that a measureable amount of silicone remains on the hair after twelve washing and rinsing cycles. Phrases such as "deposits durably" and "durable deposition" are to be interpreted accordingly.

The term HLB value is known to the skilled person working in this technical area - see for example Römpp Chemie Lexikon, Thieme Verlag, Stuttgart, $9^{th}$ Edition, 1995 under "HLB-Wert".

## DETAILED DESCRIPTION

All cited references are incorporated herein by reference in their entireties.

All percentages given herein are by weight of total composition unless specifically stated otherwise. All ratios given herein are weight ratios unless specifically stated otherwise.

All molecular weights given herein are weight average molecular weights, unless stated otherwise.

Except where specific examples of actual measured values are presented, numerical values referred to herein should be considered to be qualified by the word "about".

In examining how to solve the above technical problems, the present inventors moved away from focusing exclusively on molecular properties and started also to consider what effect altering physical properties of silicones might have. That is because we observed that silicone droplets tend to interact with strands of hair predominantly as fluids and not as individual molecules. A number of parameters were investigated and matched against the objectives.

We identified that, within a defined hydrophilicity range for the hydrophilic functionalized silicone and a defined hydrophilicity range for the hydrophobic functionalized silicone, which is immiscible with the hydrophilic functionalized silicone, advantageous technical benefits can be achieved as regards the evenness of silicone deposition and the durability of the silicone deposition on hair. While not wishing to be bound by theory, it is believed that the hydrophilic functional silicone component preferentially migrates to the aqueous interface of the blend decreasing the interfacial tension and thereby improving the deposition onto the more polar chemically damaged hair. This is also believed to lead to a more uniform coverage of the hydrophobic component for enhanced durability. Surprisingly, these benefits are found to apply to all functionalized silicone blends in which the hydrophilic and hydrophobic components are immiscible and adhere to the defined hydrophilicty ranges, regardless of chemistry of those functionalized silicones, i.e. regardless of the functional groups concerned.

Hydrophilicity has traditionally been measured by means of interfacial tension (IFT) which is conventionally established using a pendant drop-type method, as defined hereinbelow. The present inventors also used such a method as far as they were able. During the course of our investigations it became apparent, however, that the accuracy of the pendant drop method drops off significantly for functionalized silicones having interfacial tensions of less than 1 mN/m (1 dyne/cm). This is because the difference in surface energy is so low that the "drop" becomes hard to distinguish from the surrounding medium. Extremely hydrophilic silicones such as Wetsoft CTW, for example, from Wacker Silicones, is so hydrophilic that an IFT measurement using the pendant drop method is extremely difficult to perform. Unfortunately, included in the region of IFT less than 1 mN/m are hydrophilic silicones of interest to the present inventors. As a result, the present inventors were forced to adopt an alternative method for the hydrophilic functionalized silicones - the so-called hydrophilicity index (HI) as also defined hereinbelow.

The hydrophilic functionalized silicone component according to the invention has a hydrophilicity index in the range of greater than or equal to 85 and less than 100, preferably of greater than or equal to 87 and less than or equal to 99.5, more preferably of greater than or equal to 90 and less than or equal to 98.

The hydrophobic functional silicone component has an interfacial tension of less than or equal to 15 mN/m and a hydrophicity index of less than or equal to 95, preferably an interfacial tension of less than or equal to 12 mN/m and a hydrophilicity index of less than or equal to 93, more preferably an interfacial tension of less than or equal to 8 mN/m and a hydrophilicity index of less than or equal to 90.

For the sake of completeness, an IFT of 1 mN/m (1 dyne/cm) corresponds to an HI of approximately 85. For ease of comprehension, the lower the IFT value, the higher the corresponding HI value and vice versa.

The present inventors have also established that within the given hydrophilicity level of the hydrophobic functionalized silicone, the fluid viscosity has an influence on the absolute deposition level, the level of durability and also the tactile sensorial feel of the deposited silicone. Advantageously, according to an embodiment of the invention, the

hydrophobic functionalized silicone has a viscosity in the range 400 - 150,000 mPa.s. More advantageously, the viscosity is in the range 4000 - 25,000 mPa.s.

Within the given hydrophilicity range, the deposition and durability of the hydrophobic functionalized silicone increases with increasing viscosity up to a plateau viscosity which was found to be above 400 mPa.s. While not wishing to be bound by theory, at this plateau viscosity the silicone is believed to provide sufficient resistance to contraction, "roll-up" and subsequent removal during the time frame of the rinsing process to improve deposition.

We have also established that above 4000 mPa.s the tactile feel of the deposited hydrophobic functionalized silicone is improved. Without being limited by theory, we believe this is due to the formation of a smoother morphology of the deposited structures versus the fluids between 400 and 4000 mPa.s.

The viscosity of the hydrophilic functionalized silicone may range from 50 to 150,000 mPa.s, preferably from 400 to 125,000 mPa.s and more preferably from 600 to 100,000mPa.s.

According to a further advantageous embodiment of the invention, the blend of hydrophilic functionalized silicone and hydrophobic functionalized silicone is present in an amount ranging from 0.1 to 20wt%, preferably from 0.50 to 10wt% of the fiber treatment composition.

According to a further advantageous embodiment of the invention, from 1 to 90wt%, preferably from 3 to 50wt% of the functionalized silicone blend consists of hydrophilic functionalized silicone. Additionally, from 10 to 99wt%, preferably from 50 to 97wt% of the functionalized silicone blend may advantageously consist of hydrophobic functionalized silicone.

The silicone blend comprised within the fiber treatment composition according to the invention may comprise more than one hydrophilic functionalized silicone having a hydrophilicity index (HI) value of greater than or equal to 85 and less than 100, and/or more than one hydrophobic functionalized silicone having an interfacial tension (IFT) of less than or equal to 15mN/m and an HI of less than 95, provided that the hydrophilic functionalized silicones are immiscibe with the hydrophobic functionalized silicones in each case. In addition, the silicone blend may also comprise may also other functional and non-functional silicone components, which are neither hydrophilic nor hydrophobic, as defined.

The functional silicone hydrophilic and hydrophobic components may be blended either within the formula or pre-blended as a pre-mix prior to inclusion within the formula. Preferably, the functional silicone hydrophilic and hydrophobic components are pre-blended as a pre-mix prior to inclusion within the formula creating an oil-in-oil-in-water multiple emulsion, wherein the term oil is understood to comprise the hydrophilic and hydrophobic functionalized silicone components of the present invention.

Hydrophilic and hydrophobic functionalized silicones which may be incorporated into compositions according to the invention include organomodified silicones of the pendant or graft type wherein polar functional substituents are incorporated within or onto monovalent organic groups, $A^1$, $A^2$, $A^3$ and $A^4$ used hereinafter, as follows:

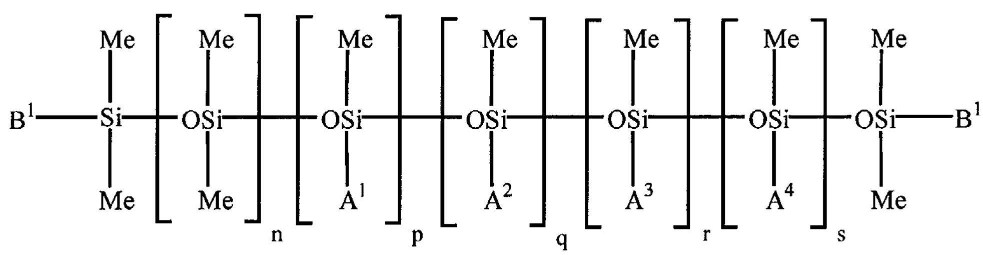

Also included are the organomodified silicones of the block copolymer type wherein these polar functional substituents are incorporated within or onto bivalent organic groups, $A^1$, $A^2$, $A^3$ and $A^4$ used hereinafter.

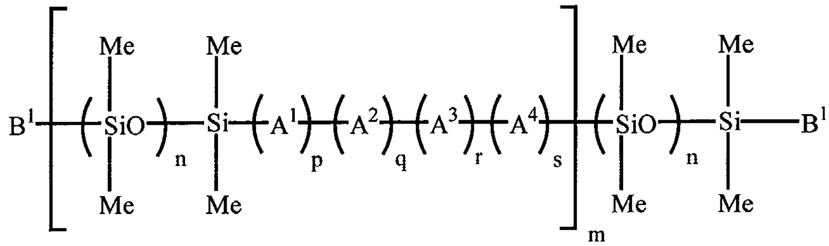

where Me is methyl, m is greater than or equal to 1, n is about 50 to 2000, p is about 0 to 50, q is about 0 to 50, r is about 0 to 50, s is about 0 to 50, wherein p + q + r + s is greater than or equal to 1, $B^1$ is H, OH, an alkyl or an alkoxy group.

The above organomodified silicones of the pendant or block copolymer type can also incorporate silicone branching groups including $MeSiO_{3/2}$, known as silsesquioxane or T groups, and $SiO_{4/2}$, known as Q groups by those skilled in the art.

Organic groups $A^1$, $A^2$, $A^3$ and $A^4$ may be straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms, especially 0, N, S, P and can incorporate one or more polar substituents selected from electron withdrawing, electron neutral, or electron donating groups with Hammett sigma para values between -1.0 and +1.5 which can be non-ionic, zwitterionic, cationic or anionic comprising, for example, groups $\alpha^1$, $\alpha^2$, $\alpha^3$, and $\alpha^4$ as defined below; S-linked groups including $S\alpha^1$, SCN, $SO_2\alpha^1$, $SO_3\alpha^1$, $SS\alpha 1^1$, $SO\alpha^1$, $SO_2N\alpha^1\alpha^2$, $SN\alpha^1\alpha^2$, $S(N\alpha^1)\ \alpha^2$, $S(O)(N\alpha^1)\alpha^2$, $S\alpha^1(N\alpha^2)$, $SON\alpha^1\alpha^2$; O-linked groups including $O\alpha^1$, $OO\alpha^1$, OCN, $ON\alpha^1\alpha^2$; N-linked groups including $N\alpha^1\alpha^2$, $N\alpha^1\alpha^2\alpha^3+$, NC, $N\alpha^1O\alpha^2$, $N\alpha^1S\alpha^2$, NCO, NCS, $NO_2$, $N{=}N\alpha^1$, $N{=}NO\alpha^1$, $N\alpha^1CN$, $N{=}C{=}N\alpha^1$, $N\alpha^1N\alpha^2\alpha^3$, $N\alpha^1N\alpha^2N\alpha^3\alpha^4$, $N\alpha^1N{=}N\alpha^2$; other miscellaneous groups including COX, $CON_3$, $CON\alpha^1\alpha^2$, $CON\alpha^1CO\alpha^2$, $C({=}N\alpha^1)N\alpha^1\alpha^2$, CHO, CHS, CN, NC, and X.

$\alpha^1$, $\alpha^2$, $\alpha^3$, and $\alpha^4$ may be straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms, especially 0, N, S, P.

X is F, Cl, Br, or I.

H is hydrogen, O is oxygen, N is nitrogen, C is carbon, S is sulfur, Cl is chlorine, Br is bromine, I is iodine, F is fluorine.

Hammett sigma para values are discussed in Römpp Chemie Lexikon, Georg Thieme Verlag, Stuttgart, New York, 9th Edition, 1995 under "Hammett Gleichung".

In the case of the hydrophilic functionalized silicone, preferred polar functional substituents include, but are not limited to, polyoxyalkylene (polyether), primary and secondary amine, amide, quaternary ammonium, carboxyl, sulfonate, sulfate, carbohydrate, phosphate, and hydroxyl. More preferably, the polar functional substituents of the present invention include, but are not limited to polyoxyalkylene, primary and secondary amine, amide and carboxyl.

Preferred polar functional substituents for inclusion within the hydrophilic functionalized silicone contain at least one class of oxygen containing polar functional substituent, such that the oxygen content (% Oxygen) within the summation of the one or more polar functional substituents (not including the oxygen in the PDMS backbone) is from 1% to 17%, preferably from 2% to 15%, and more preferably from 3% to 13% of the weight of the functionalized silicone. In addition, the hydrophilic functional silicone components of the present invention should have a silicone content (% Silicone) of from 45 to 95%, preferably from 50 to 90%, and more preferably from 55 to 85% of the weight of the functionalized silicone. The silicone content or calculated percent silicone (% Silicone) is defined as the average molecular weight of the PDMS backbone (consisting of silicon, oxygen and, where present, methyl groups) divided by the average molecular weight of the whole polymer. Similarly, the overall oxygen content (% Oxygen) is defined as the molecular weight of each oxygen atom multiplied by the average number of oxygen atoms present on the silicone and then divided by the average molecular weight of the whole polymer.

More preferably, the hydrophilic functionalized silicone polymer comprises polyoxyalkylene substituents. The polyoxyalkylene content (% polyether) should be from 5 to 55%, preferably from 10 to 50%, and more preferably from 15 to 45%. Preferably, the sum of % silicone and % polyether does not total 100%, other constituents, such as amine and amide making up the balance. The silicone content is defined above and the polyether content (% polyether) is defined as the molecular weight of each polyether pendant or block multiplied by the average number of pendants or blocks and divided by the average molecular weight of the whole polymer. If the pendant or block polyether comprises of both ethylene oxide (EO) and propylene oxide (PO) units, then this % polyether comprises the summation of % EO and % PO. If the pendant or block polyether is comprised of either only EO or only PO units, this % polyether is equivalent to the %EO or %PO, respectively.

More preferably still, the hydrophilic functionalized silicone is according to the following formula (1):

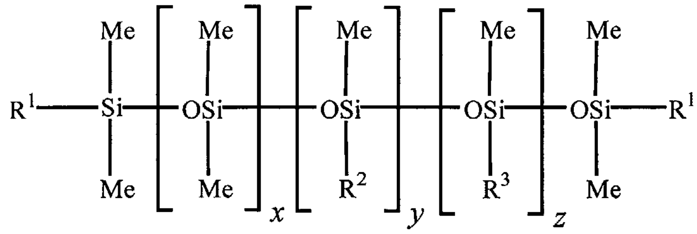

(1)

where Me equals methyl; $R^1$ is methyl or $R^2$ or $R^3$; $R^2$ is $-(CH_2)_a-NH-[(CH_2)_a-NH]_b-H$; and $R^3$ is $-(CH_2)_a-(OC_2H_4)_m-(OC_3H_6)_n-OZ$; wherein x is about 50 to 1500, y is about 1 to 20, z is about 1 to 20; a is about 2 to 5, preferably 2 to 4; b is 0 to 3, preferably 1; m is about 1 to 30; n is about 1 to 30, and Z is H, an alkyl group with 1-4 carbons, or an acetyl group, with the proviso that when y is 0, $R^1$ is an $R^2$ group, and when z is 0, $R^1$ is an $R^3$ group.

The pendant organomodified silicones comprising amino and polyoxyalkylene groups of the average formula (1) can be prepared by methods known to those skilled in the art, via steps including known polymerisation reactions (e.g. equilibration or polycondensation) and known methods of introducing organic substitution to the silicone backbone (e.g. hydrosilitation).

The following are non-limiting exemplary structures of the hydrophilic functionalized silicone according to the present invention.

Material A

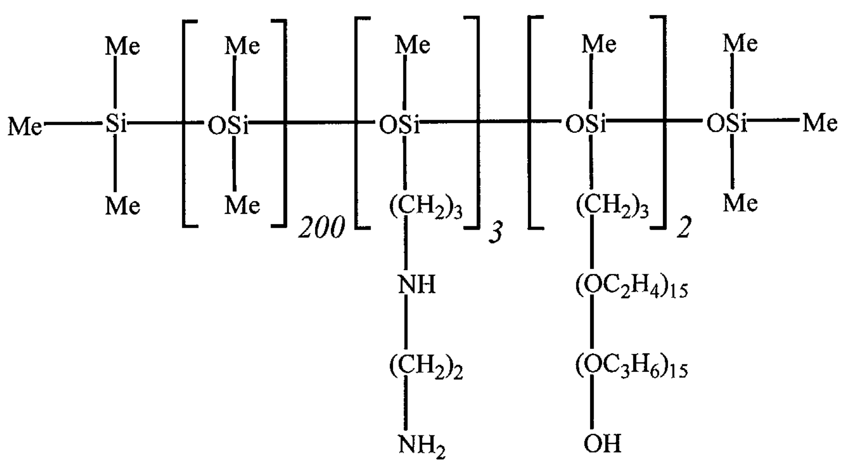

Material B

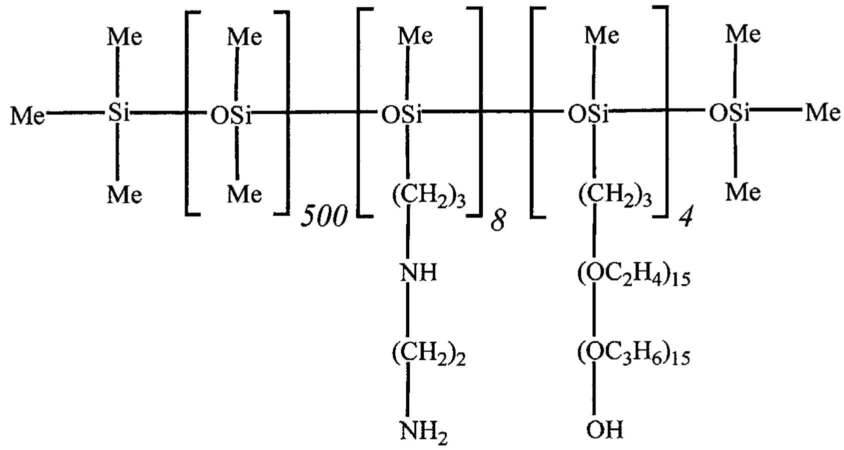

Material C

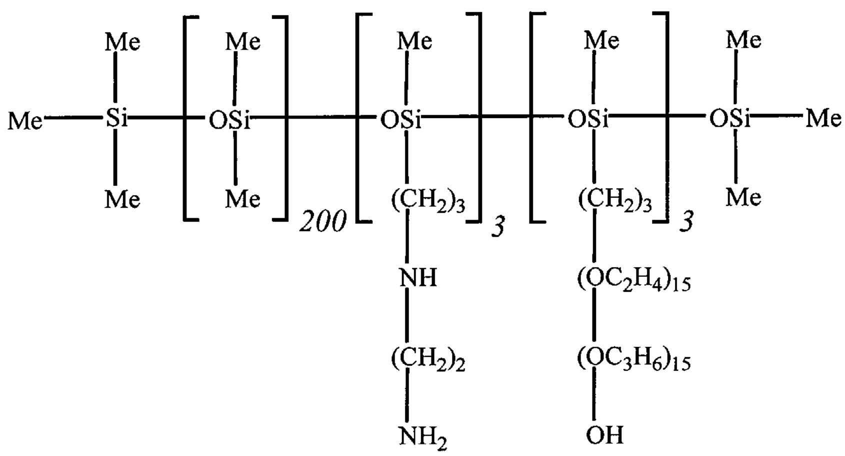

Material D

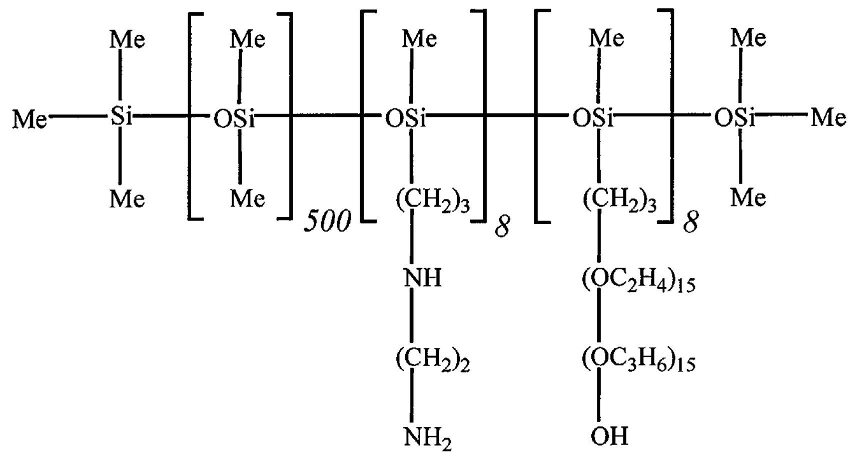

Preferred polar functional substituents for use with the hydrophobic functionalized silicone according to the present invention include, but are not limited to, polyoxyalkylene (polyether), primary and secondary amine, amide, quaternary ammonium, carboxyl, sulfonate, sulfate, carbohydrate, phosphate, and hydroxyl. More preferably, the polar functional substituents of the present invention include, but are not limited to polyoxyalkylene, primary and secondary amine, amide and carboxyl.

Suitable hydrophobic functionalized silicones according to the present invention include, but are not limited to, organomodified silicones with amine functionality available commercially under the trade names such as ADM1100 and ADM1600 from Wacker Silicones, DC2-8211, DC8822, DC8822A, DC8803, DC2-8040, DC2-8813, DC2-8630 and DC8566 from Dow Corning Corporation, KF-862, KF-861, KF-862S, KF-8005, KF-8004, KF-867S, KF-873, and X-52-2328 from Shin-Etsu Corporation, and TSF 4702, TSF 4703, TSF 4704, TSF 4705, TSF 4707, TSF 4708, TSF 4709, F42-B3115, SF 1708, SF 1923, SF 1921, SF 1925, OF TP AC3309, OF 7747, OF-NH TP AI3631, OF-NH TP AI3683 from GE Bayer Silicones.

Preferred hydrophobic functionalized silicones include organomodified silicones with amine functionality and viscosities of greater than 4,000 mPa.s. These include, but are not limited to, commercially available fluids under the trade names ADM1100 from Wacker Silicones, DC8803 from Dow Corning Corporation, and TSF 4707 from GE Bayer Silicones.

The fiber treatment composition according to the invention is advantageously a hair treatment composition. In such a case, the composition may additionally comprise a hair bleaching component and/or a hair dyeing component.

According to a further aspect of the invention, a hair treatment kit is provided comprising:

(a) an oxidative bleaching composition
(b) a dye composition

a hair treatment composition as defined hereinabove comprised within component (a) and/or within component (b) and/or provided as a separate component.

The fiber treatment composition according to the present invention may include a cosmetically acceptable vehicle to act as a diluent, dispersant, or carrier for the silicone oil in the composition, so as to facilitate the distribution of the silicone oil when the composition is applied. The vehicle may be an aqueous emulsion, water, liquid or solid emollients, solvents, humectants, propellants, thickeners and powders.

Advantageously, the fiber treatment compositions according to the present invention may be in the form an emulsion with water as a primary component, although aqueous organic solvents may also be included. The emulsion is preferably an oil-in-oil-in-water (silicone-in-silicone-in-water) emulsion.

The aqueous continuous phase of the emulsion treatment compositions of the present invention may further comprise an emulsifier to facilitate the formation of the emulsion. Emulsifiers for use in the aqueous continuous phase

of the present emulsion treatment compositions may include an anionic surfactant, cationic surfactant, amphoteric surfactant, water-soluble polymeric surfactant, water-soluble silicone-containing surfactant, nonionic surfactant having an HLB of greater than about 10, or a surfactant system capable of forming stabilizing liquid crystals around the silicone droplets. The nonionic surfactant preferably has an HLB of at least 12, and more preferably, an HLB value of at least about 15. Surfactants belonging to these classes are listed in McCutcheon's Emulsifiers and Detergents, North American and International Editions, MC Publishing Co., Glen Rock NJ, pages 235-246 (1993).

The emulsifier for the aqueous phase does not gel the aqueous phase. The emulsifier however may be capable of forming a stabilizing layer of lamellar liquid crystals around silicone droplets. This barrier film prevents coalescence between emulsion droplets. In this case, the surfactant system can be a single surfactant or a blend of surfactants. In some cases, a particular surfactant cannot form a liquid crystal structure alone, but can participate in the formation of liquid crystals in the presence of a second surfactant. Such a surfactant system forms a layer of lamellar liquid crystals around the silicone to provide a barrier between the silicone and the aqueous phase. This type of an emulsion is different from the conventional emulsions, which rely upon the orientation of the hydrophobic and hydrophilic components of a surfactant at an silicone-water interface. The formation of a layer of lamellar liquid crystals around the silicone can be detected by the presence of Maltese crosses viewed by optical microscopy through crossed polarizing plates or by freeze fracture electron microscopy.

Exemplary classes of surfactants capable of participating in the formation of a liquid crystal structure around the silicone droplets include, but are not limited to specific cationic surfactants, anionic surfactants, nonionic surfactants, quaternary ammonium surfactants and lipid surfactants.

Preferred surfactants for the formation of liquid crystals in the aqueous continuous phase are of the nonionic type and include $C_{16-22}$ fatty alcohols, and $C_{16-22}$ fatty alcohol ethoxylates with 1 to 30 ethylene oxide groups. Specific examples include cetearyl alcohol, cetyl alcohol, stearyl alcohol, arachidyl alcohol, oleyl alcohol, ceteareth ethoxylates with between 10 and 30 ethylene oxide groups, ceteth ethoxylates with between 10 to 30 ethylene oxide groups, steareth ethoxylates with between 10 and 30 ethoxylates, and combinations thereof. Preferably, $C_{16-22}$ fatty alcohols are used in combination with $C_{16-22}$ fatty alchol ethoxylates at a ratio of between 10:1 to 0.5:1, more preferably between 6:1 and 1:1, and most preferably between 5:1 and 1.5:1.

The aqueous continuous phase should ideally comprise the emulsifier in an amount sufficient to stabilize the silicone. In one embodiment, the aqueous continuous phase comprises the emulsifier in an amount of from about 0.1% to about 15%, and more preferably from about 0.1% to about 10%, based on the weight of the aqueous continuous phase.

The composition according to the present application finds particular utility in hair coloring compositions especially oxidative hair colorants wherein the hair is subjected to a particularly aggressive environment.

A preferred hair coloring agent for use herein is an oxidative hair coloring agent. The concentration of each oxidative hair coloring agent in the compositions according to the present invention may be from about 0.0001% to about 5% by weight.

Any oxidative hair coloring agent can be used in the compositions herein. Typically, oxidative hair coloring agents comprise at least two components, which are collectively referred to as dye forming intermediates (or precursors). Dye forming intermediates can react in the presence of a suitable oxidant to form a colored molecule.

The dye forming intermediates used in oxidative hair colorants include: aromatic diamines, aminophenols, various heterocycles, phenols, napthols and their various derivatives. These dye forming intermediates can be broadly classified as; primary intermediates and secondary intermediates. Primary intermediates, which are also known as oxidative dye precursors, are chemical compounds which become activated upon oxidation and can then react with each other and/or with couplers to form colored dye complexes. The secondary intermediates, also known as color modifiers or couplers, are generally colorless molecules which can form colors in the presence of activated precursors/primary intermediates, and are used with other intermediates to generate specific color effects or to stabilise the color.

Primary intermediates suitable for use in the compositions and processes herein include: aromatic diamines, polyhydric phenols, amino phenols and derivatives of these aromatic compounds (e.g., N-substituted derivatives of the amines, and ethers of the phenols). Such primary intermediates are generally colorless molecules prior to oxidation.

While not wishing to be bound by any particular theory, it is believed that the process by which color is generated from these primary intermediates and secondary coupler compounds generally includes a stepwise sequence whereby the primary intermediate can become activated (by oxidation), and then enjoins with a coupler to give a dimeric, conjugated colored species, which in turn can enjoin with another 'activated' primary intermediate to produce a trimeric conjugated colored molecule.

In general terms, oxidative dye primary intermediates include those materials which, on oxidation, form oligomers or polymers having extended conjugated systems of electrons in their molecular structure. Because of the new electronic structure, the resultant oligomers and polymers exhibit a shift in their electronic spectra to the visible range and appear colored. For example, oxidative primary intermediates capable of forming colored polymers include materials such as aniline, which has a single functional group and which, on oxidation, forms a series of conjugated imines

and quinoid dimers, trimers, etc. ranging in color from green to black. Compounds such as p-phenylenediamine, which has two functional groups, are capable of oxidative polymerization to yield higher molecular weight colored materials having extended conjugated electron systems. Oxidative dyes known in the art can be used in the compositions according to the present invention. A representative list of primary intermediates and secondary couplers suitable for use herein is found in Sagarin, "Cosmetic Science and Technology"," Interscience, Special Ed. Vol. 2 pages 308 to 310.

The primary intermediates can be used alone or in combination with other primary intermediates, and one or more can be used in combination with one or more couplers. The choice of primary intermediates and couplers will be determined by the color, shade and intensity of coloration which is desired. There are nineteen preferred primary intermediates and couplers which can be used herein, singly or in combination, to provide dyes having a variety of shades ranging from ash blonde to black; these are: pyrogallol, resorcinol, p-toluenediamine, p-phenylenediamine, o-phenylenediamine, m-phenylenediamine, o-aminophenol, p-aminophenol, 4-amino-2-nitrophenol, nitro-p-phenylenediamine, N-phenyl-p-phenylenediamine, m-aminophenol, 2-amino-3-hydroxypyridine, 1-napthol, N,N bis (2-hydroxyethyl)p-phenylenediamine, resourcinol, diaminopyrazole, 4-amino-2-hydroxytoluene, 1,5-dihydroxynapthalene, 2-methyl resorcinol and 2,4-diaminoanisole. These can be used in the molecular form or in the form of peroxide-compatible salts.

The hair coloring compositions of the present invention may, in addition to or instead of an oxidative hair coloring agent, include non-oxidative and other dye materials. Optional non-oxidative and other dyes suitable for use in the hair coloring compositions and processes according to the present invention include both semi-permanent, temporary and other dyes. Non-oxidative dyes as defined herein include the so-called 'direct action dyes', metallic dyes, metal chelate dyes, fiber reactive dyes and other synthetic and natural dyes. Various types of non-oxidative dyes are detailed in: 'Chemical and Physical Behaviour of Human Hair' 3rd Ed. by Clarence Robbins (pp250-259); 'The Chemistry and Manufacture of Cosmetics'. Volume IV. 2nd Ed. Maison G. De Navarre at chapter 45 by G.S. Kass (pp841-920); 'cosmetics: Science and Technology' 2nd Ed., Vol. II Balsam Sagarin, Chapter 23 by F.E. Wall (pp 279-343); 'The Science of Hair Care' edited by C. Zviak, Chapter 7 (pp 235-261) and .'Hair Dyes', J.C. Johnson, Noyes Data Corp., Park Ridge, U.S.A. (1973), (pp 3-91 and 113-139).

The hair coloring compositions herein preferably comprise at least one oxidising agent, which may be an inorganic or organic oxidising agent. The oxidising agent is preferably present in the coloring composition at a level of from about 0.01% to about 10%, preferably from about 0.01% to about 6%, more preferably from about 1% to about 4% by weight of the composition.

A preferred oxidising agent for use herein is an inorganic peroxygen oxidising agent. The inorganic peroxygen oxidising agent should be safe and effective for use in the present compositions. Preferably, the inorganic peroxygen oxidising agents suitable for use herein will be soluble in the compositions according to the present invention when in liquid form or in the form intended to be used. Preferably, inorganic peroxygen oxidising agents suitable for use herein will be water-soluble. Water soluble oxidising agents as defined herein means agents which have a solubility to the extent of about 10g in 1000ml of deionised water at 25°C ("Chemistry" C. E. Mortimer. 5th Edn. p277).

The inorganic peroxygen oxidising agents useful herein are generally inorganic peroxygen materials capable of yielding peroxide in an aqueous solution. Inorganic peroxygen oxidising agents are well known in the art and include hydrogen peroxide, inorganic alkali metal peroxides such as sodium periodate, sodium perbromate and sodium peroxide, and inorganic perhydrate salt oxidising compounds, such as the alkali metal salts of perborates, percarbonates, perphosphates, persilicates, persulphates and the like. These inorganic perhydrate salts may be incorporated as monohydrates, tetrahydrates etc. Mixtures of two or more of such inorganic peroxygen oxidising agents can be used if desired. While alkali metal bromates and iodates are suitable for use herein the bromates are preferred. Highly preferred for use in the compositions according to the present invention is hydrogen peroxide.

The compositions herein may instead or in addition to the inorganic peroxygen oxidising agent(s), comprise one or more preformed organic peroxyacid oxidising agents.

Suitable organic peroxyacid oxidising agents for use in the coloring compositions according to the present invention have the general formula:

$$R\text{-}C(O)OOH$$

wherein R is selected from saturated or unsaturated, substituted or unsubstituted, straight or branched chain, alkyl, aryl or alkaryl groups with from 1 to 14 carbon atoms.

The organic peroxyacid oxidising agents should be safe and effective for use in the compositions herein. Preferably, the preformed organic peroxyacid oxidising agents suitable for use herein will be soluble in the compositions used according to the present invention when in liquid form and in the form intended to be used. Preferably, organic peroxyacid oxidising agents suitable for use herein will be water-soluble. Water-soluble preformed organic peroxyacid oxidising agents as defined herein means agents which have a solubility to the extent of about 10g in 1000ml of deionised water at 25°C ("Chemistry" C. E. Mortimer. 5th Edn. p277).

The compositions herein may optionally contain a transition metal containing catalyst for the inorganic peroxygen oxidising agents and the optional preformed peroxy acid oxidising agent(s). Suitable catalysts for use herein are disclosed in WO98/27945.

The compositions herein may contain as an optional component a heavy metal ion sequestrant. By heavy metal ion sequestrant it is meant herein components which act to sequester (chelate or scavenge) heavy metal ions. These components may also have calcium and magnesium chelation capacity, but preferably they show selectivity to binding heavy metal ions such as iron, manganese and copper. Such sequestering agents are valuable in hair coloring compositions as herein described for the delivery of controlled oxidising action as well as for the provision of good storage stability of the hair coloring products.

Heavy metal ion sequestrants may be present at a level of from about 0.005% to about 20%, preferably from about 0.01% to about 10%, more preferably from about 0.05% to about 2% by weight of the compositions.

Suitable sequestering agents are disclosed in WO98/27945.

For use, the treatment compositions according to an embodiment of the invention may be provided at a pH from about 3 to 11, preferably from 4 to 10.5.

The present compositions do not only find application in the treatment of fibers, such as hair, but may also be applied to other substrates, such as human skin, nails and various animal body parts, such as horns, hooves and feathers.

## TEST METHODS

### Hydrophilicity Index Method:

The hydrophilicity indexes were measured via turbidimetry on an LP2000 Turbidity Meter from Hanna Instruments, Bedfordshire, United Kingdom.

A 100ml beaker is thoroughly cleaned, including prior rinsing with hexane then ethanol, and then dried:

1. 0.3500 grams (+/- 0.0015g) of the functionlized silicone is weighed directly into the beaker.

2. 24.6500 grams (+/-0.0500g) of ethanol (ethyl alcohol, 99.7% vol/vol minimum, A.R. quality, EEC No. 200-578-6) is then weighed directly into the beaker.

3. The contents of the beaker is then mixed thoroughly via a high shear mixer (IKA Labortechnik - Ultra - Turrax T8 from IKA Werke GmbH & Co. KG, Staufen, Germany) for 1 minute with special attention to ensure the silicone is completely removed from the bottom of the beaker and thereby mixed properly.

4. Immediately dispense using a pipette, 10ml of the resulting liquid into a clean cuvette (Note: The cuvette is thoroughly rinsed with hexane twice, then rinsed with ethanol twice and then dried prior to readings) which is then loaded into the turbidity meter.

5. After approximately 30 seconds a turbidity reading is recorded, immediately followed by a second reading for verification.

6. Steps 1-5 are repeated 3 times, the turbidity values being averaged to give the average turbidity reading for the functionalized silicone.

The hydrophilicity index for the functionalized silicone is then computed as follows:

$$\text{Hydrophilicity Index} = 100 - ((\text{Average turbidity})/400)\times 100$$

### Interfacial tension measurement protocol

The silicone/water interfacial tensions of the organomodified silicones were measured via pendant drop shape analysis on a Kruss DSA-10 instrument as taught in F. K. Hansen, G. Rodsrun, "Surface tension by pendant drop. A fast standard instrument using computer image analysis", Journal of Colloid and Interface Science, Volume 141, Issue 1, January 1991, pages 1-9. The accuracy of this method is dependent upon the density difference between the reference fluid (usually water) and the test fluid. Given that many of the present functionalized silicones have densities

approaching that of water, $D_2O$ (with a density of 1.1 g/$cm^3$) was substituted for $H_2O$ as the more dense phase, in order to ensure a sufficient density difference. The respective densities of the organomodified silicones were measured with a Calculating Precision Density Meter DMA 55 instrument from Apollo Scientific Limited.

Viscosity of functionalized silicone fluids - measurement protocol

An AR 500 rotational rheometer (TA Instruments Ltd., Leatherhead, Surrey KT22 7UQ, UK) is used to determine the viscosity of the functionalized silicone fluids used herein. The determination is performed at 30°C, with the 4cm 2° steel cone measuring system set with a 49µm (micron) gap and is performed via the programmed application of a shear stress of 0.5 to 590 Pa over a 2 minute time period. These data are used to create a shear rate vs. shear stress curve for the material. This flow curve can then be modelled in order to provide a material's viscosity. These results were fitted with the following well-accepted Newtonian model:

$$\text{Viscosity, } \mu = \sigma/\gamma$$

(where σ is shear stress; y is shear rate)

Method for assessing silicone particle size within a product

A microscope (Nikon Eclipse E800) is utilised to determine the silicone particle size in the final product. Typically, pictures are taken (JVC color video camera KY-F50) of the final product at a magnification ranging from 100x to 400x. Using the captured image a scale is superimposed (Image software - Lucia G Version 4.51 (build 028), Laboratory Imaging) previously calibrated using a 100 µm Graticule (Graticules Ltd, Tonbridge Wells, Kent, England) and compared to the average silicone particle within the sample to provide an estimation of particle size.

EXAMPLES

The following examples further describe and demonstrate the preferred embodiments within the scope of the present invention. The examples are given solely for the purpose of illustration, and are not to be construed as limitations of the present invention since many variations thereof are possible without departing from its scope.

Examples 1-4 - Colorant compositions

| **Peroxide base** | **#1** | **#2** | **#3** | **#4** |
|---|---|---|---|---|
| Ingredients | Wt% | Wt% | Wt% | Wt% |
| Emulsion base: | | | | |
| Deionized water | 29.17 | 29.17 | 29.17 | 29.17 |
| Cetyl alcohol (1) | 2.20 | 2.20 | 2.20 | 2.20 |
| Stearyl alcohol (2) | 2.20 | 2.20 | 2.20 | 2.20 |
| Ceteareth-25 (3) | 1.47 | 1.47 | 1.47 | 1.47 |
| Phenoxyethanol (4) | 0.11 | 0.11 | 0.11 | 0.11 1 |
| Sodium benzoate (5) | 0.09 | 0.09 | 0.09 | 0.09 |
| Tetrasodium EDTA (87%) (6) | 0.04 | 0.04 | 0.04 | 0.04 |
| | | | | |
| | | | | |
| Deionized water | 35.00 | 35.00 | 35.00 | 35.00 |
| Pentasodium pentetate (40%) (7 | 0.24 | 0.24 | 0.24 | 0.24 |
| Hydroxyethane diphosphonic acid (60%) (8) | 0.16 | 0.16 | 0.16 | 0.16 |

(continued)

| Peroxide base | #1 | #2 | #3 | #4 |
|---|---|---|---|---|
| Ingredients | Wt% | Wt% | Wt% | Wt% |
| Emulsion base: | | | | |
| Phosphoric acid (75%) (9) | 0.08 | 0.08 | 0.08 | 0.08 |
| Sodium stannate (95%) (10) | 0.04 | 0.04 | 0.04 | 0.04 |
| | | | | |
| Hydrogen peroxide (35%) (11) | 16.80 | 16.80 | 16.80 | 16.80 |
| Deionized water | 10.40 | 10.40 | 9.40 | 9.40 |
| | | | | |
| Silicone premix: | | | | |
| -Methoxy PEG/PPG-7/3 aminopropyl dimethicone sold under the name Abil soft AF100 by the company Goldschmidt | | | 0.5 | |
| -Aminopolyether functional silicone fluid sold under the name XS69-B5476 by the company GE bayer silicones | 0.50 | 1.00 | | 0.50 |
| - Aminofunctional polydimethylsiloxane sold under the name Wacker-belsil®ADM1100 by the company Wacker | | | | 1.50 |
| -Aminofunctional polydimethylsiloxane sold under the name Dowcorning 2-8566 silicone fluids by the company Dow corning | 1.50 | 1.00 | 1.50 | |

| Carrier base for dye base | #1 | #2 | #3 | #4 |
|---|---|---|---|---|
| Ingredients | Wt% | Wt% | Wt% | Wt% |
| | | | | |
| Deionized water | 46.49 | 46.49 | 46.49 | 46.49 |
| Acetic acid (50%) (12) | 3.91 | 3.91 | 3.91 | 3.91 |
| Emulsion base (see ingredients above) | 36.00 | 36.00 | 36.00 | 36.00 |
| Ammonium hydroxide (13) | 13.60 | 13.60 | 13.60 | 13.60 |

(1): available as Surfac cetyl alcohol from Surfachem, Leeds, UK
(2): available as Surfac stearyl alcohol from Surfachem, Leeds, UK
(3): available as Volpo CS25 from Croda, North Humberside. UK
(4): available as Phenoxyethanol from Nipa-Hardwicke, Wilmington, Delaware
(5): available as Sodium benzoate EP/USP from Haltermann, Cumbria, UK
(6): available as Edeta B powder from BASF, Cheadle, Cheshire, UK
(7): available as Trilon C liquid from BASF, Cheadle, Cheshire, UK
(8): available as Dequest 2010 from Solutia, Newport, South wales
(9): available as Phosphoric acid 750F from Albright & Wilson, West Midlands, UK
(10): available as Sodium stannate, Aldrich
(11): available as Hydrogen peroxide 35% 171/4 from Ellis & Everard, Walsall, UK
(12): available as 50% acetic acid from Hays, Greenwich, London, UK
(13): available as Ammonium Solution BP grade from Brotherton Speciality Products, West Yorkshire, UK

Production of the example colorant applications

Peroxide base:

The emulsion base is prepared by adding to a vessel the deionized water and commencing agitation with heating to 82°C. Then the preservatives (tetrasodium EDTA, sodium benzoate) are added and dissolved. This is followed by addition of ceteareth25, cetyl alcohol and stearyl alcohol while keeping the temperature above 80°C. Then phenoxytol is added. The mixture is then fully blended hot through a recirculation line and homogenized. The emulsion structure is obtained by cooling the product down below 50°C and shearing while cooling. The product is left to thicken for 60min.

The chelant premix is prepared by adding the chelants to water and mixing them together in a vessel. Then this solution is added to the emulsion base. The completed peroxide base is made by adding water to the previous mixture followed by the hydrogen peroxide while stirring.

The two functionalized silicones are pre-mixed together under agitation and then added to the peroxide base and stirred until the desired particle size is obtained.

Carrier system for dye base :

The carrier base is prepared by adding water to a vessel and commencing agitation, followed by the addition of acetic acid. Then emulsion base (see emulsion base preparation described above) is added. When fully homogenized, ammonium hydroxide is added to the mixture.

For application to hair the peroxide base and the dye base are mixed together at a 1:1 ratio and applied to dry hair.

Examples 5-7 - After colorant conditioners

| | **#5** | **#6** | **#7** |
|---|---|---|---|
| Ingredients | Wt% | Wt% | Wt% |
| | | | |
| Deionized water | 61.95 - qs | 60.95 - qs | 61.95 - qs |
| | | | |
| Emulsion base: | | | |
| Deionized water | 29.76 | 29.76 | 29.76 |
| Cetyl alcohol (1) | 2.25 | 2.25 | 2.25 |
| Stearyl alcohol (2) | 2.25 | 2.25 | 2.25 |
| Ceteareth-25 (3) | 1.50 | 1.50 | 1.50 |
| Phenoxyethanol (4) | 0.11 | 0.11 | 0.11 |
| Sodium benzoate (5) | 0.09 | 0.09 | 0.09 |
| Tetrasodium EDTA (87%) (6) | 0.04 | 0.04 | 0.04 |
| | | | |
| Citric acid anhydrous fine (14) | pH trim | pH trim | pH trim |
| Silicone premix: | | | |
| -Aminofunctional polydimethylsiloxane sold under the name Wacker-belsil®ADM1100 by the company Wacker | 1.50 | 0 | 0 |
| -Aminopolyether functional silicone fluid sold under the name TSF4707 by the company GE bayer silicones | 0 | 0 | 1.50 |

(continued)

| | #5 | #6 | #7 |
|---|---|---|---|
| Silicone premix: | | | |
| -Aminofunctional polydimethylsiloxane sold under the name Dowcorning 2-8566 silicone fluids by the company Dow corning | 0 | 2.25 | 0 |
| -Aminopolyether functional silicone fluid sold under the name XS69-B5476 by the company GE bayer silicones | 0.50 | 0.75 | 0.50 |
| (14): available as citric acid anhydrous fine from Aldrich | | | |

Composition preparation

The conditioner composition is prepared by adding to a vessel the deionized water and the emulsion base (see emulsion base preparation described above) while stirring. When homogenized citric acid is added to the mixture until the pH of the emusltion is between 5 and 6.

The functionalised silicones premix is prepared by pre-mixing together the two functionalised silicone fluids with agitation. The functionalised silicone premix is then added to the main mix and stirred until the desired particle size is obtained.

**Claims**

1. Fiber treatment composition comprising a blend of a hydrophilic functionalized silicone having a hydrophilicity index (HI) value of greater than or equal to 85 and less than 100, and a hydrophobic functionalized silicone having an interfacial tension (IFT) of less than or equal to 15mN/m and an HI of less than 95, wherein the hydrophilic silicone and the hydrophobic silicone are immiscible with one another.

2. Fiber treatment composition according to claim 1, wherein the hydrophilic functionalized silicone has an HI in the range $87 \leq HI \leq 99.5$.

3. Fiber treatment composition according to claim 1 or 2, wherein the hydrophobic functionalized silicone has an IFT less than or equal to 12 mN/m and an HI of less than or equal to 93.

4. Fiber treatment composition according to any one of the preceding claims, wherein the viscosity of the hydrophobic functionalized silicone is in the range 50 to 150,000 mPa.s, preferably in the range 400 to 125,000 mPa.s.

5. Fiber treatment composition according to any one of the preceding claims, wherein the viscosity of the hydrophobic functionalized silicone is in the range 4000 to 100,000 mPa.s.

6. Fiber treatment composition according to any one of the preceding claims, wherein the functionalized silicone blend deposits evenly and durably on the fibers being treated.

7. Fiber treatment composition according to any one of the preceding claims, wherein the blend of hydrophilic functionalized silicone and hydrophobic functionalized silicone is present in an amount ranging from 0.1 to 20wt%, preferably from 0.50 to 10wt%.

8. Fiber treatment composition according to any one of the preceding claims, wherein from 1 to 90wt%, preferably from 3 to 50wt% of the functionalized silicone blend consists of hydrophilic functionalized silicone.

9. Fiber treatment composition according to any one of the preceding claims, wherein from 10 to 99wt%, preferably from 50 to 97wt% of the functionalized silicone blend consists of hydrophobic functionalized silicone.

10. Fiber treatment composition according to any one of the preceding claims which is in the form of an silicone-in-silicone-in-water emulsion.

11. Fiber treatment composition according to claim 10 additionally comprising 0.1 to 15% based on the weight of the

aqueous continuous phase of emulsifier.

**12.** Fiber treatment composition according to claim 11, wherein the emulsifier comprises one or more of an anionic surfactant, cationic surfactant, amphoteric surfactant, water-soluble polymeric surfactant, water soluble silicone-containing surfactant and a non-ionic surfactant.

**13.** Fiber treatment composition according to claim 12, wherein the surfactant comprises $C_{16}$ - $C_{22}$ fatty alcohols and/or fatty alcohol ethoxylates with 1 to 30 ethylene oxide groups, preferably 10 to 30 ethylene oxide groups.

**14.** Fiber treatment composition according to claim 13, wherein the sufactant comprises a mixture of $C_{16-22}$ fatty alcohols and $C_{16-22}$ fatty alchol ethoxylates in a ratio of between 10:1 to 0.5:1, more preferably between 6:1 and 1:1.

**15.** Fiber treatment composition according to any one of the preceding claims, wherein both the hydrophilic and the hydrophobic functionalized silicones are organomodified silicones of the pendant or graft type according to the following formula:

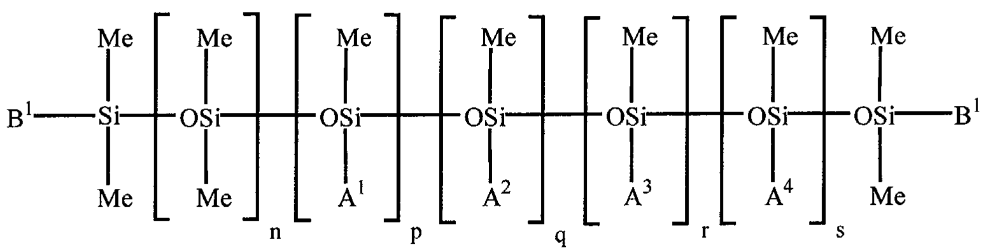

or a block copolymer type according to the following formula:

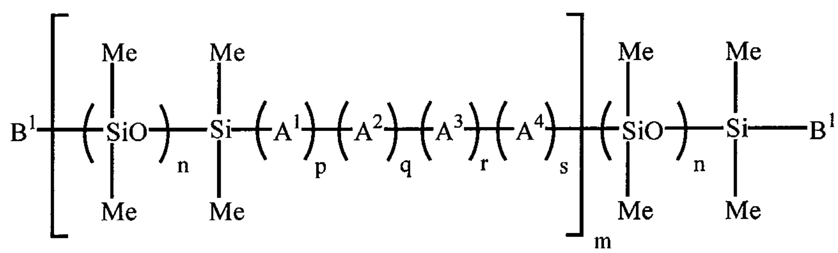

where Me is methyl, m is greater than or equal to 1, n is about 50 to 2000, p is about 0 to 50, q is about 0 to 50, r is about 0 to 50, s is about 0 to 50, wherein p + q + r + s is greater than or equal to 1, $B^1$ is H, OH, an alkyl or an alkoxy group and organic groups $A^1$, $A^2$, $A^3$ and $A^4$ are straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms incorporating one or more polar substituents selected from electron withdrawing, electron neutral, or electron donating groups with Hammett sigma para values between -1.0 and +1.5.

**16.** Fiber treatment composition according to claim 15, wherein the polar substituents comprise groups $\alpha^1$, $\alpha^2$, $\alpha^3$, and $\alpha^4$ as defined below; S-linked groups including $S\alpha^1$, SCN, $SO_2\alpha^1$, $SO_3\alpha^1$, $SS\alpha 1^1$, $SO\alpha^1$, $SO_2N\alpha^1\alpha^2$, $SN\alpha^1\alpha^2$, S $(N\alpha^1)$ $\alpha^2$, $S(O)(N\alpha^1)$ $\alpha^2$, $S\alpha^1(N\alpha^2)$, $SON\alpha^1\alpha^2$; O-linked groups including $O\alpha^1$, $OO\alpha^1$, OCN, $ON\alpha^1\alpha^2$; N-linked groups including $N\alpha^1\alpha^2$, $N\alpha^1\alpha^2\alpha^3$+, NC, $N\alpha^1O\alpha^2$, $N\alpha^1S\alpha^2$, NCO, NCS, $NO_2$, $N{=}N\alpha^1$, $N{=}NO\alpha^1$, $N\alpha^1CN$, $N{=}C{=}N\alpha^1$, $N\alpha^1N\alpha^2\alpha^3$, $N\alpha^1N\alpha^2N\alpha^3\alpha^4$, $N\alpha^1N{=}N\alpha^2$; COX, $CON_3$, $CON\alpha^1\alpha^2$, $CON\alpha^1CO\alpha^2$, $C({=}N\alpha^1)N\alpha^1\alpha^2$, CHO, CHS, CN, NC, and X, where:

$\alpha^1$, $\alpha^2$, $\alpha^3$, and $\alpha^4$ are straight, branched or mono- or polycyclic aliphatic, mono or polyunsaturated alkyl, aryl, heteroalkyl, heteroaliphatic or heteroolefinic moiety comprising 3 to 150 carbon atoms together with 0-50 heteroatoms, especially 0, N, S, P, and

X is F, Cl, Br, or I, where

H is hydrogen, O is oxygen, N is nitrogen, C is carbon, S is sulfur, Cl is chlorine, Br is bromine, I is iodine, F is fluorine.

**17.** Fiber treatment composition according to either of claims 15 or 16, wherein, in the case of the hydrophilic functionalized silicone, the one or more polar substituents comprise oxygen, such that the oxygen contents of the summation of the one or more polar substituents is from 1% to 17% of the weight of the functionalized silicone and the silicone content is from 45 to 95% of the weight of the functionalized silicone.

**18.** Fiber treatment composition according to any one of claims 15 to 17, wherein the hydrophilic functionalized silicone comprises polyoxyalkylene substituents.

**19.** Fiber treatment composition according to claim 18, wherein the hydrophilic functionalized silicones are according to the following formula:

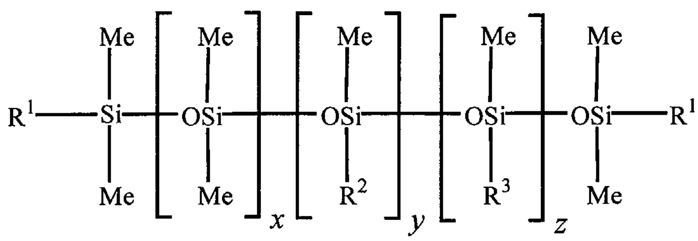

where Me equals methyl; $R^1$ is methyl or $R^2$ or $R^3$; $R^2$ is $-(CH_2)_a-NH-[(CH_2)_a-NH]_b-H$; and $R^3$ is $-(CH_2)_a-(OC_2H_4)_m-(OC_3H_6)_n-OZ$; wherein x is about 50 to 1500, y is about 1 to 20, z is about 1 to 20; a is about 2 to 5, preferably 2 to 4; b is 0 to 3, preferably 1; m is about 1 to 30; n is about 1 to 30, and Z is H, an alkyl group with 1-4 carbons, or an acetyl group, with the proviso that when y is 0, $R^1$ is an $R^2$ group, and when z is 0, $R^1$ is an $R^3$ group.

**20.** Fiber treatment composition according to claim 18 or 19, wherein the polyoxyalkylene content is from 5 to 42% and the silicone content is from 67 and 95%.

**21.** Fiber treatment composition according to any one of the preceding claims comprising a hydrophilic functionalized silicone selected from materials A to D below and mixtures of these materials:
Material A

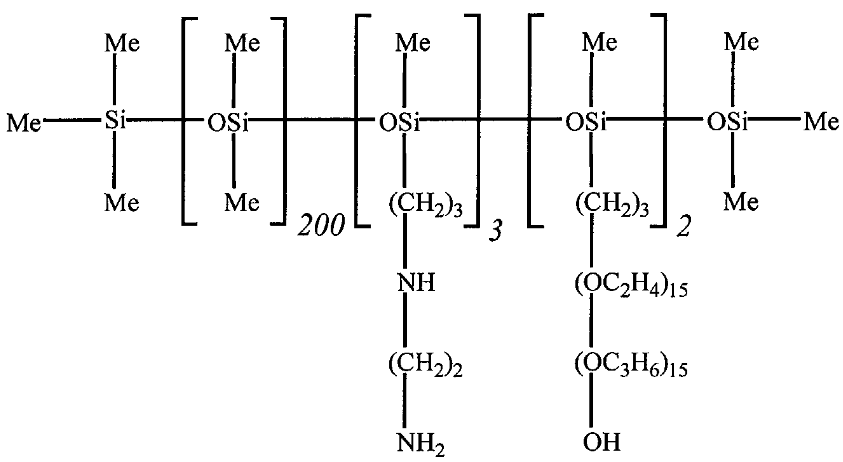

Material B

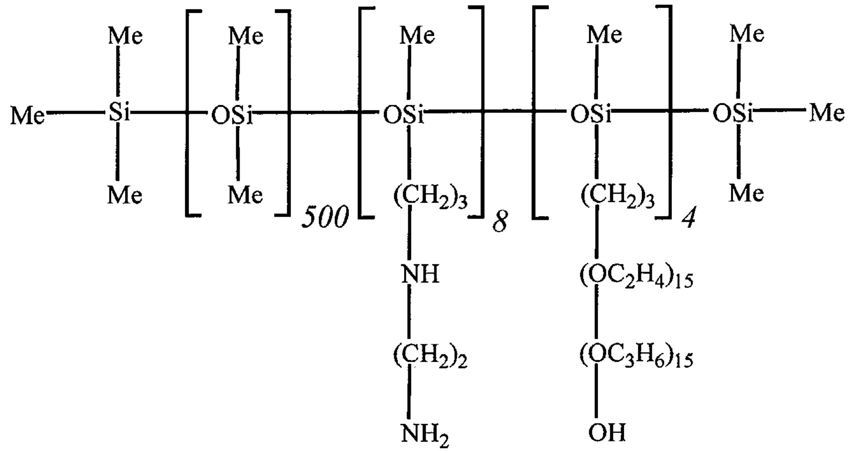

Material C

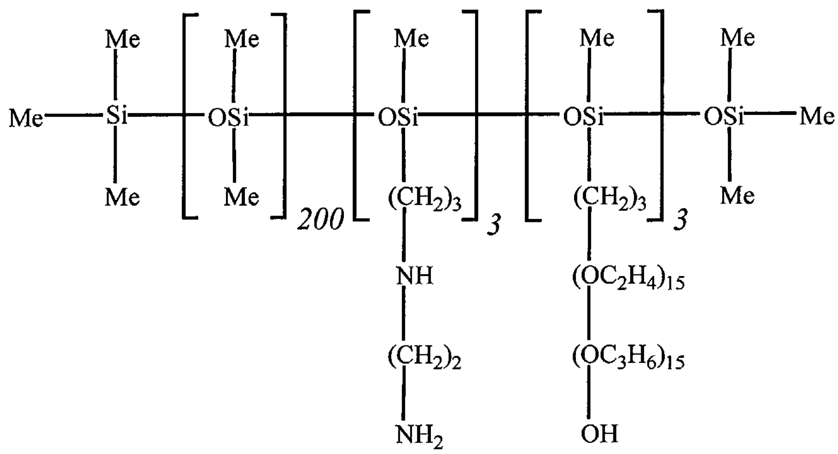

Material D

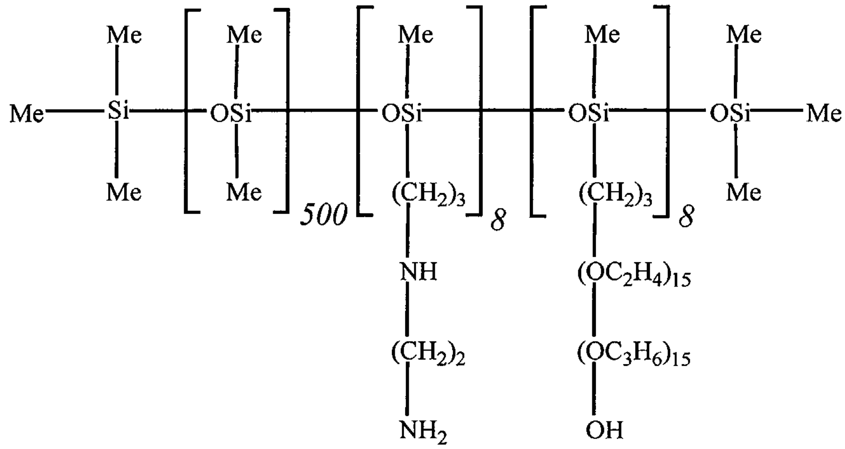

**22.** Fiber treatment composition according to either of claims 15 or 16, wherein, in the case of the hydrophobic functionalized silicone, the polar substituents are selected from polyoxyalkylene, primary and secondary amine, amide, quaternary ammonium, carboxyl, sulfonate, sulfate, carbohydrate, phosphate, and hydroxyl and mixtures of these.

**23.** Fiber treatment composition according to claim 22, wherein the polar substituents comprise amine substituents.

**24.** Fiber treatment composition according to any one of the preceding claims, wherein the fiber is hair.

**25.** Hair treatment composition according to claim 24 additionally comprising a hair bleaching component and/or a hair dyeing component.

**26.** Hair treatment kit comprising:

(a) an oxidative bleaching composition
(b) a dye composition; and

a hair treatment composition according to claim 24 comprised within component (a) and/or within component (b) and/or provided as a separate component.

European Patent Office

# PARTIAL EUROPEAN SEARCH REPORT

which under Rule 45 of the European Patent Convention shall be considered, for the purposes of subsequent proceedings, as the European search report

Application Number EP 03 25 2204

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 081 272 A (DOW CORNING CORP.) 7 March 2001 (2001-03-07)<br>* the whole document *<br>--- | 1-26 | A61K7/06<br>D06M15/643 |
| X | A. CZECH ET AL.: "Modified silicone softeners for fluorocarbon soil release treatments"<br>BOOK OF PAPERS- INTERNATIONAL CONFERENCE & EXHIBITION,AMERICAN ASSOCIATION OF TEXTILE CHEMISTS AND COLORISTS,<br>1996, pages 230-238, XP001164104<br>USA<br>* the whole document *<br>--- | 1-26 | |
| A | WO 93 07848 A (THE PROCTER & GAMBLE CO.) 29 April 1993 (1993-04-29)<br>* claims 1,9; example 2 *<br>--- | 1-26 | |
| X | WO 02 22084 A (THE PROCTER & GAMBLE CO.) 21 March 2002 (2002-03-21)<br>* claim 1; example 2 *<br>---<br>-/-- | 1-26 | TECHNICAL FIELDS SEARCHED (Int.Cl.7)<br>A61K<br>A61Q |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 6 August 2003 | Glikman, J-F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH**
**SHEET C**

Application Number
EP 03 25 2204

Claim(s) searched incompletely:
1-26

Reason for the limitation of the search:

Present claims 1-26 relate to a composition and a kit defined (inter alia) by reference to the following parameters:
P1: hydrophilicity index
P2: interfacial tension
P3: Hammett sigma para values (claim 15)

The use of these parameters in the present context is considered to lead to a lack of clarity within the meaning of Article 84 EPC. It is impossible to compare the parameters the applicant has chosen to employ with what is set out in the prior art. The lack of clarity is such as to render a meaningful complete search impossible.

Present claim 6 moreover relates to a composition defined by reference to a desirable characteristic or property, namely "the functionalised silicone blend deposits evenly and durably on the fibres being treated" The claim covers all compositions having this characteristic or property, whereas the application provides support within the meaning of Article 84 EPC and/or disclosure within the meaning of Article 83 EPC for only a very limited number of such compositions. In the present case, the claims so lack support, and the application so lacks disclosure, that a meaningful search over the whole of the claimed scope is impossible. Independent of the above reasoning, the claim also lacks clarity (Article 84 EPC). An attempt is made to define the composition by reference to a result to be achieved. Again, this lack of clarity in the present case is such as to render a meaningful search over the whole of the claimed scope impossible.

Consequently, the search has been carried out for those parts of the claims which appear to be clear, supported and disclosed, namely those parts relating to the compositions and kits comprising a hydrophilic functionalised silicone and a hydrophobic functionalised silicone wherein the hydrophilic functionalised silicone is to be chosen among silicones according to the formula p.14, paragraph 3 to p.15, paragraph 1 of the description (present claim 19) and the hydrophobic functionalised silicone is to be chosen among the silicones as described on p.17, paragraph 1 of the description ( present claim 22).

European Patent Office

# PARTIAL EUROPEAN SEARCH REPORT

Application Number
EP 03 25 2204

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 99 29286 A (UNILEVER PLC ET AL.) 17 June 1999 (1999-06-17) * the whole document * --- | 1 | |
| A | EP 1 175 893 A (KAO CORP.) 30 January 2002 (2002-01-30) * the whole document * ----- | 25,26 | |
| | | | TECHNICAL FIELDS SEARCHED (Int.Cl.7) |

EPO FORM 1503 03.82 (P04C10)

## ANNEX TO THE EUROPEAN SEARCH REPORT ON EUROPEAN PATENT APPLICATION NO. EP 03 25 2204

This annex lists the patent family membersrelating to the patent documents cited in the above-mentioned European search report.
The members are as contained in the European Patent Office EDP file on
The European Patent Office is in no way liable for these particulars which are merely given for the purpose of information.

06-08-2003

| Patent document cited in search report | Publication date | Patent family member(s) | Publication date |
|---|---|---|---|
| EP 1081272 A | 07-03-2001 | US 6136215 A | 24-10-2000 |
| | | US 6255429 B1 | 03-07-2001 |
| | | EP 1081272 A1 | 07-03-2001 |
| | | JP 2001115031 A | 24-04-2001 |
| | | JP 2001089571 A | 03-04-2001 |
| WO 9307848 A | 29-04-1993 | US 5277899 A | 11-01-1994 |
| | | AU 2783892 A | 21-05-1993 |
| | | CA 2117264 A1 | 29-04-1993 |
| | | EP 0663813 A1 | 26-07-1995 |
| | | JP 7500334 T | 12-01-1995 |
| | | WO 9307848 A2 | 29-04-1993 |
| WO 0222084 A | 21-03-2002 | WO 0222089 A1 | 21-03-2002 |
| | | AU 7479900 A | 26-03-2002 |
| | | AU 9080301 A | 26-03-2002 |
| | | AU 9262401 A | 26-03-2002 |
| | | CA 2422048 A1 | 21-03-2002 |
| | | EP 1317240 A2 | 11-06-2003 |
| | | WO 0222084 A2 | 21-03-2002 |
| | | WO 0222085 A2 | 21-03-2002 |
| WO 9929286 A | 17-06-1999 | AU 735831 B2 | 19-07-2001 |
| | | AU 2154199 A | 28-06-1999 |
| | | BR 9813380 A | 03-10-2000 |
| | | CA 2309950 A1 | 17-06-1999 |
| | | CN 1280484 T | 17-01-2001 |
| | | WO 9929286 A1 | 17-06-1999 |
| | | EP 1035827 A1 | 20-09-2000 |
| | | JP 2001525352 T | 11-12-2001 |
| | | PL 340916 A1 | 12-03-2001 |
| | | US 2001056048 A1 | 27-12-2001 |
| EP 1175893 A | 30-01-2002 | JP 2002037718 A | 06-02-2002 |
| | | EP 1175893 A2 | 30-01-2002 |

EPO FORM P0459

For more details about this annex : see Official Journal of the European Patent Office, No. 12/82